# EUROPEAN PATENT SPECIFICATION

(11) **EP 1 685 130 B1**
(45) Date of publication and mention of the grant of the patent: **10.12.2008**
(21) Application number: 04791330.6
(22) Date of filing: 28.10.2004
(51) Int. Cl.: C07D 471/04, A61K 31/437, A61P 31/04

(54) **POLYMORPHS OF 1-CYCLOPROPYL-7-([S,S] - 2,8-DIAZADICYCLO [4.3.0] NON-8-YL) -6-FLUORO-1,4-DIHYDRO-8-METHOXY-4-OXO-3-QUINOLINE CARBOXYLIC ACID HYDROCHLORIDE AND METHODS FOR THE PREPARATION THEREOF**
POLYMORPHE VON 1-CYCLOPROPYL-7-([S,S] - 2,8-DIAZADICYCLO [4.3.0] NON-8-YL) -6-FLUOR-1,4-DIHYDRO-8-METHOXY-4-OXO-3-CHINOLINCARBONSÄUREHYDROCHLORID UND VERFAHREN ZU DEREN HERSTELLUNG
POLYMORPHES D'HYDROCHLORURE D'ACIDE 1-CYCLOPROPYL-7-([S,S]-2,8-DIAZADICYCLO[4.3.0]NON-8-YL)-6-FLUORO-1,4-DIHYDRO-8-METHOXY-4-OXO-3-QUINOLINE CARBOXYLIQUE, ET LEURS METHODES DE PREPARATION

(30) Priority: 20.11.2003 IT MI20032259; 24.12.2003 US 532779 P
(43) Date of publication of application: 02.08.2006
(73) Proprietor: CHEMI S.p.A., 20092 Cinisello Balsamo (Milano) (IT)
(72) Inventor: TURCHETTA, Stefano, I-00139, Roma (IT); AROMATARIO, Valentina, I-00177 Roma (IT)
(74) Representative: Pistolesi, Roberto
(86) International application number: PCT/EP2004/052699
(87) International publication number: WO 2005/054240

(56) References cited:
- EP-A- 0 550 903
- EP-A- 0 780 390
- WO-A-03/049688
- WO-A-20/04091619

## Description

### Field of the invention

The present invention relates to two novel polymorphs of 1-cyclopropyl-7-([S,S]-2,8-diazadicyclo[4.3.0]-non-8-yl)-6-fluoro-1,4-dihydro-8-methoxy-4-oxo-3-quinoline carboxylic acid hydrochloride, the methods for the preparation thereof, and pharmaceutical formulations which include them.

### State of the art

1-cyclopropyl-7-([S,S]-2,8-diazadicyclo[4.3.0]non-8-yl)-6-fluoro-1,4-dihydro-8-methoxy-4-oxo-3-quinoline carboxylic acid hydrochloride, also known by the name moxifloxacin hydrochloride, is an antibacterial agent of formula:

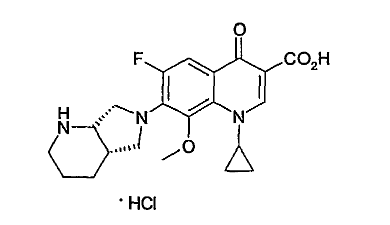

which is widely used therapeutically in the treatment of infections by antibiotic-resistant bacteria.

Its preparation is reported in EP550903 and the preparation and characteristics of its monohydrate pseudopolymorph are described in US5849752. It is clear from this patent that the only known forms of moxifloxacin hydrochloride are the anhydrous form and the monohydrate, extensive identifying documentation of which is provided. In the patent, it is also written that the anhydrous form of the active ingredient is unsuitable for the preparation of pharmaceutical formulations since it absorbs water from the atmosphere. The monohydrate form, on the other hand, does not have the disadvantage of being hygroscopic and can be prepared in the form of crystalline prisms, which confer on the powder characteristics of fluidity that are advantageous for formulation purposes, or in the form of needles which tend rather to clump together.

According to the US patent, the monohydrate in prism form can be produced by suspending moxifloxacin hydrochloride in ethanol/water mixtures containing up to 10% of water whereas, to form the monohydrate in needle form, water or any water/alcohol mixture with a water content greater than 10% may be used.

It is also mentioned in the description of the invention that, in order to produce the monohydrate form, the relative humidity value should not fall below 30% during the drying stage, since this condition would lead to the formation of the anhydrous form.

However, the examples of the preparation of moxifloxacin hydrochloride monohydrate given in US5849752 show serious limitations of industrial applicability both owing to the large volumes of solvent that are used and owing to the subsequent production technique.

In fact the method provides first of all for the anhydrous form of moxifloxacin hydrochloride to be dissolved in a large quantity of solvent and then for the solvent to be evaporated completely so that the active ingredient is recovered as the evaporation residue. However, if this evaporation to dryness is performed hot, for example, by heating to 60-70°C, it may lead to degradation of the product whereas, if it is performed spontaneously at room temperature as described in Examples 5 and 6 of the US patent, it requires very long periods of time that are not practicable industrially.

In conclusion, there is at the moment still a need to identify an industrially applicable method of producing a stable and easily formulated form of moxifloxacin hydrochloride which does not require laborious stages for the evaporation of large volumes of solvent and which is sufficiently quick and gentle not to lead to alterations in the final product.

### Description of the invention

The subjects of the present invention are therefore two novel, stable, and easily formulated crystalline forms of moxifloxacin hydrochloride, a method for the preparation thereof, and pharmaceutical formulations, which include them.

It has in fact surprisingly been found that, by means of an easily industrially applicable method, which comprises the steps of:
a) suspending moxifloxacin hydrochloride in a suitable solvent,
b) heating the mixture under reflux,
c) cooling, and
d) isolating the product which is separated,
a novel hydrated crystalline form of moxifloxacin hydrochloride, which is stable and easy to formulate, designated as moxifloxacin hydrochloride form A is obtained.

Further, by subjecting the product resulting form the above step d) to the following additional stages characterized by
e) reslurrying the solid in a suitable solvent, and
f) isolating the product,
another novel form of moxifloxacin hydrochloride, named form B, stable and easy to formulate too, is prepared.

The starting moxifloxacin hydrochloride may be either in amorphous form or in any crystalline form, for example, in anhydrous or monohydrate crystalline form, as described in US5849752. Preferably, the starting moxifloxacin hydrochloride is an anhydrous form having a water content of less than 0.3%.

The solvent used in the method described above is generally an alcohol or a polyol, preferably a Cₗ-C₆ alcohol or polyol, for example, methanol, ethanol, isopropanol, n-propanol, n-butanol, isobutanol, terbutanol, n-pentanol, n-hexanol, 1,2-ethandiol, 1,2-propandiol, 1,3-propandiol, methoxyethanol, methoxypropanol, etc..., most preferably ethanol or isopropanol, or mixtures thereof.

In particular, the novel crystalline forms of moxifloxacin hydrochloride can be produced, in accordance with the above-described steps, by the suspension of moxifloxacin hydrochloride in the preselected solvent, provided that the resulting mixture has an overall water content of between 2.5% and 0.01% by weight. "Overall water content" means the quantity of water resulting from the sum of the water content of the starting moxifloxacin hydrochloride and of the water contained in the solvent.

Preferably, a solvent with a water content of between 1% and 0.01%, more preferably between 0.3% and 0.01%, and even more preferably between 0.1 and 0.01% is used. The production of these novel crystalline forms is particularly surprising in the light of the misleading teaching provided by the patent US5849752; in fact in the description thereof (col. 2, lines 62-65) it is stated that "the preferred monohydrate form in the form of prisms can be obtained by suspending the crystalline anhydrous product in ethanol/water mixtures, particularly in the said mixtures with a maximum water content of 10%", thus meaning also mixtures with 2.5%, 1% or 0.1% of water. In fact, in these conditions, it has surprisingly been found that, instead of the prism form described by the US patent, form A or, following the additional steps e) - f), form B, which are the subject of the present invention, are obtained.

In the method for the preparation of form A, the solvent is generally used in a ratio of between 50:1 and 2:1, preferably between 30:1 and 5:1, more preferably about 10:1, the ratio being expressed as ml of solvent per gram of moxifloxacin hydrochloride.

The mixture of moxifloxacin hydrochloride and solvent is kept under reflux (step a) for a variable period of time which will depend on various factors such as, for example, the type of solvent, the form of the starting product, the total quantity of water, etc., and is preferably at least 1 hour, more preferably about 4 hours.

The cooling of the mixture (step b) may be spontaneous or accelerated by appropriate means known to a person skilled in the art. The mixture may be cooled to room temperature or cooling may continue to lower temperatures; in general, it is preferred to allow the mixture to cool spontaneously until room temperature is reached.

In a particularly preferred method, cooling to room temperature takes place in about 2 hours and the mixture is allowed to rest at that temperature for a further 2 hours before the isolation is performed.

The novel crystalline forms according to the invention are isolated (step d and f) by conventional techniques, for example, by filtration, decantation or centrifuging.

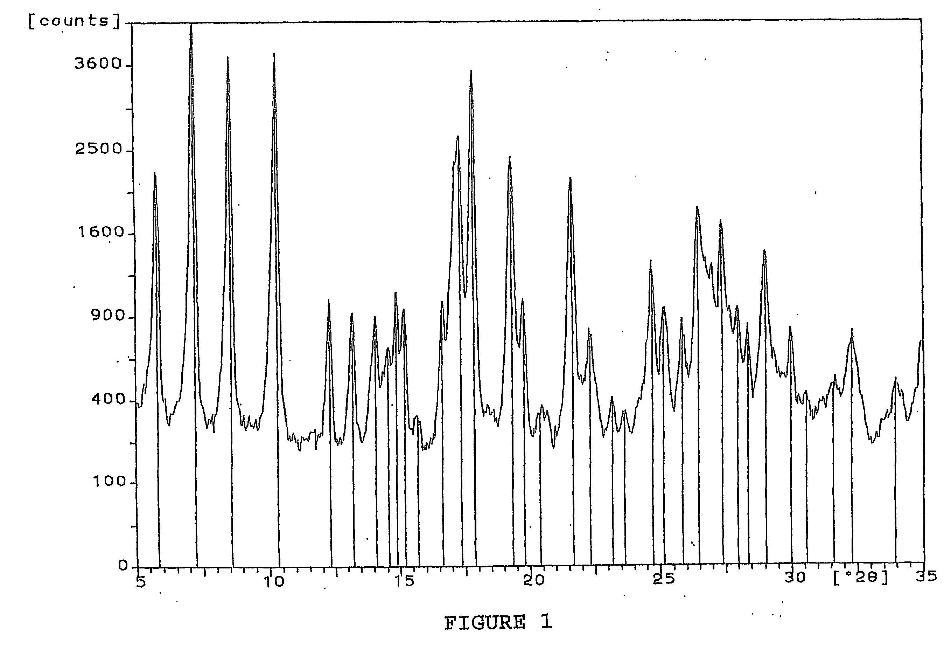

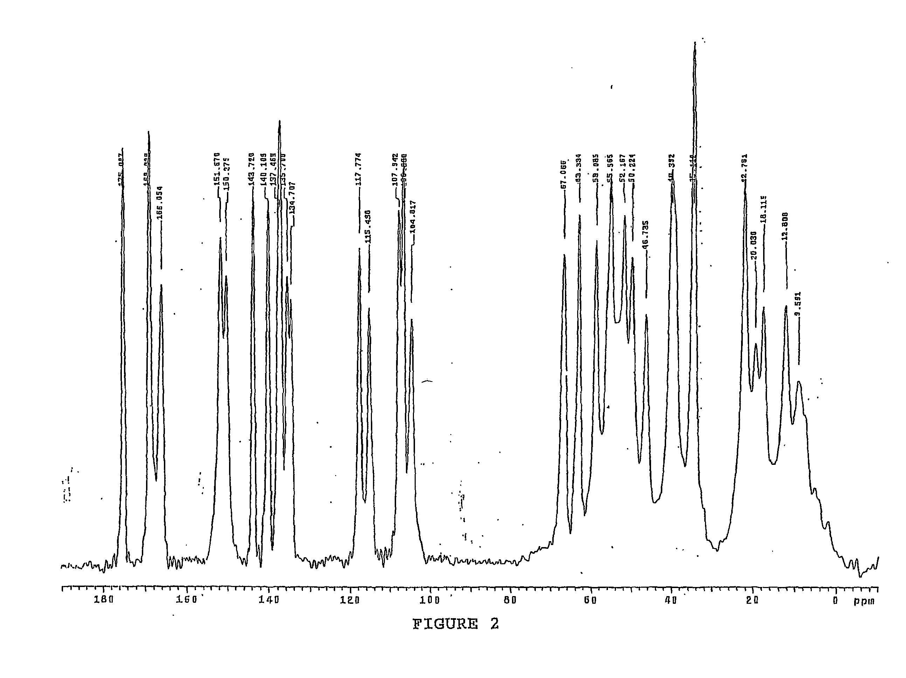

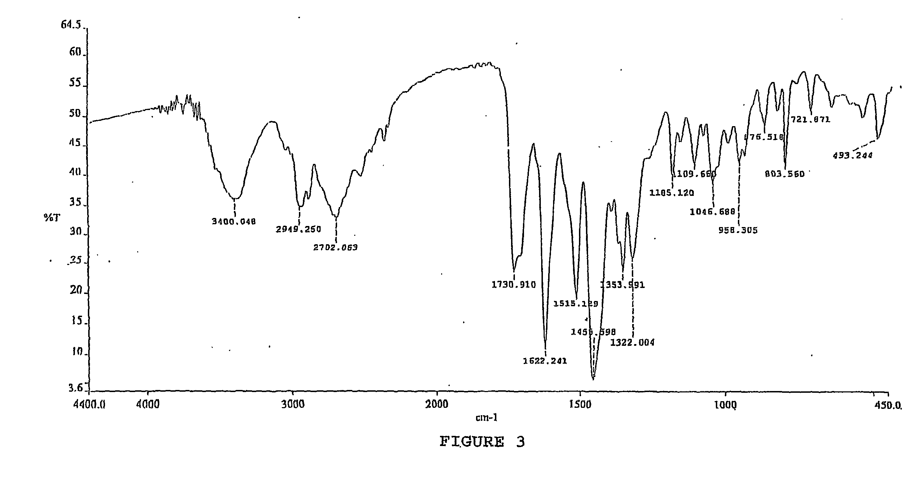

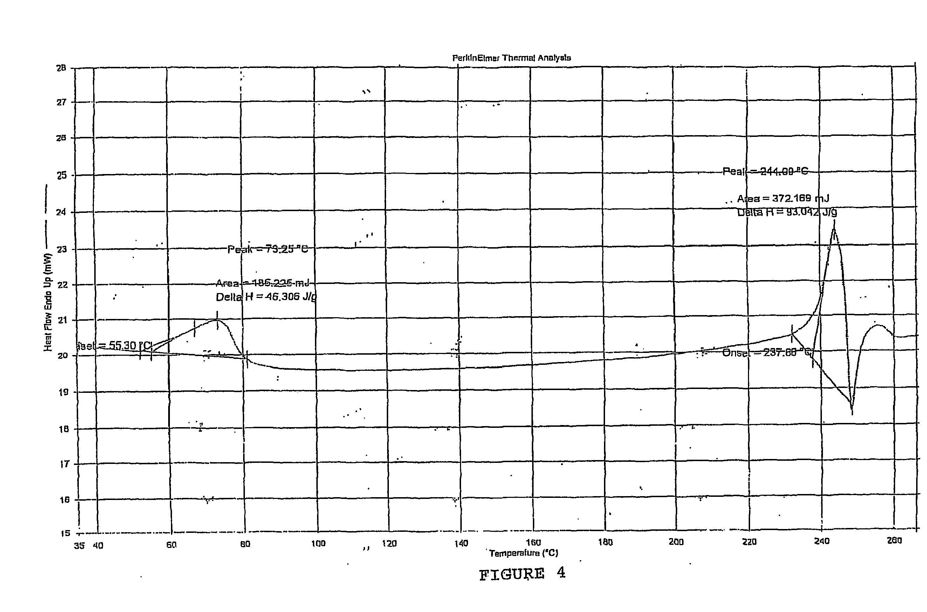

The novel crystalline form A of moxifloxacin hydrochloride is characterized by the X-ray diffraction spectrum (XRD) which is shown in Figure 1 and described in Table 1, by the solid-state ¹³C-NMR spectrum which is shown in Figure 2 and tabulated in Table 2, by the IR spectrum which is given in Figure 3, and by the DSC trace which is shown in Figure 4. The differences in comparison with the known forms can clearly be distinguished by comparing this spectral data with that of the anhydrous and monohydrate forms described in US5849752. In particular, in the XRD spectrum of the novel form, characteristic peaks situated at 7.2, 12.3, 16.6 and 21.6 are distinguished and, in the solid-state ¹³C-NMR spectrum, characteristic peaks are shown at 169.1, 164.6, 151.8, 115.7 and 67.7, as results from the following table:

**Table 1**

| X-ray diffraction spectrum of moxifloxacin hydrochloride form A, registered on a Philips PW3710 spectrometer (X-ray Diffractometer). | | |
|---|---|---|
| (Cu Ka radiation, generator voltage 40 kV, slit divergence 1°, receiving slit 0.2 mm, scan mode step start angle 5.000, end angle 35.000, time per step 2.000s) | | |
| Angle (2Θ) | D (Å) | Rel. Intens. (I/I₀) |
| 5.815 | 15.1858 | 49.8 |
| 7.220 | 12.2335 | 100.0 |
| 8.575 | 10.3032 | 86.1 |
| 10.335 | 8.5522 | 87.2 |
| 12.310 | 7.1842 | 19.4 |
| 13.200 | 6.7018 | 17.0 |
| 14.085 | 6.2826 | 16.3 |
| 14.535 | 6.0891 | 11.1 |
| 14.870 | 5.9527 | 20.6 |
| 15.185 | 5.8299 | 17.6 |
| 15.675 | 5.6487 | 1.9 |
| 16.620 | 5.3296 | 18.3 |
| 17.335 | 5.1114 | 60.1 |
| 17.850 | 4.9650 | 80.9 |
| 19.315 | 4.5916 | 53.7 |
| 19.760 | 4.4892 | 19.1 |
| 20.375 | 4.3551 | 2.5 |
| 21.640 | 4.1033 | 47.6 |
| 22.295 | 3.9842 | 12.7 |
| 23.160 | 3.8373 | 4.2 |
| 23.625 | 3.7628 | 1.9 |
| 24.705 | 3.6007 | 26.9 |
| 25.115 | 3.5428 | 17.6 |
| 25.815 | 3.4483 | 15.6 |
| 26.440 | 3.3682 | 39.4 |
| 27.365 | 3.2564 | 36.3 |
| 27.970 | 3.1874 | 17.8 |
| 28.360 | 3.1444 | 14.5 |
| 29.015 | 3.0749 | 28.2 |
| 29.965 | 2.9795 | 13.9 |
| 30.545 | 2.9243 | 4.8 |
| 31.575 | 2.8312 | 5.9 |
| 32.270 | 2.7718 | 12.2 |
| 33.900 | 2.6421 | 6.4 |

**Table 2**

| Solid-state ¹³C-NMR absorptions of moxifloxacin hydrochloride form A (p.p.m.). Spectrum registered on Varian 400 instrument: | | | | | | | | | |
|---|---|---|---|---|---|---|---|---|---|
| 175.1 | 169.0 | 166.1 | 151.7 | 150.3 | 143.7 | 140.1 | 137.5 | 135.7 | 134.7 |
| 117.8 | 115.4 | 107.9 | 106.0 | 104.8 | 67.1 | 63.3 | 59.1 | 55.6 | 52.2 |
| 50.2 | 46.7 | 40.4 | 35.1 | 22.8 | 20.0 | 18.1 | 12.8 | 9.6 | |

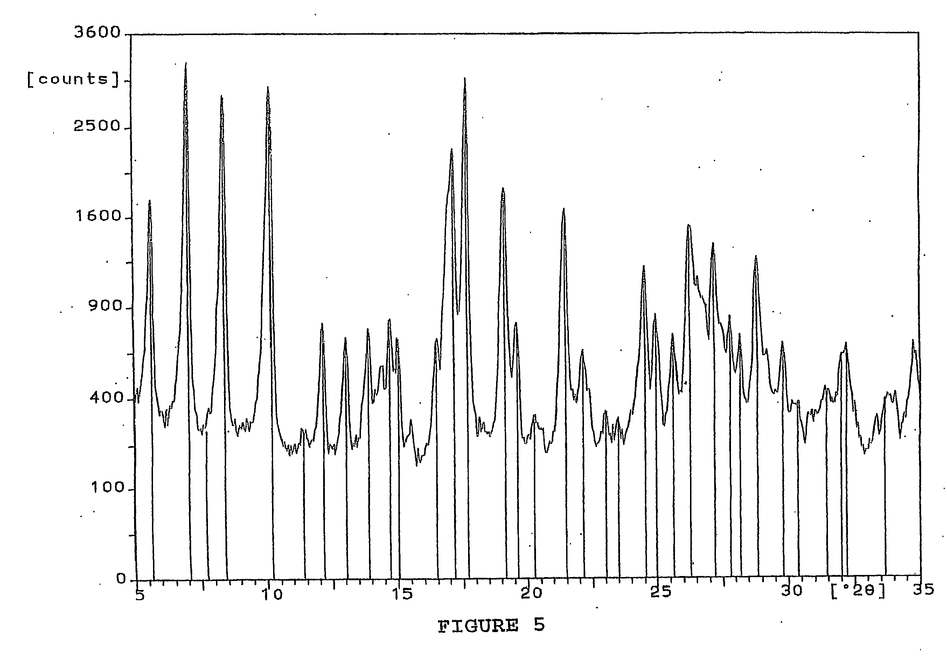

The novel crystalline form A of moxifloxacin hydrochloride is stable if left in air at room temperature for 5 days (Figure 5); in fact it can be seen from the figure that the XRD profile does not differ substantially from the reference profile of the novel form A.

Moxifloxacin hydrochloride form A has workability and fluidity characteristics, which are optimal for formulation and does not lose these properties even after compression tests.

As previously stated, Moxifloxacin hydrochloride form B may be prepared according to the following steps:
a) suspending moxifloxacin hydrochloride in a solvent selected from alcohols and polyols or mixtures thereof, in which the resulting mixture has an overall water content of between 2.5% and 0.01% by weight,
b) heating the mixture under reflux,
c) cooling,
d) filtering the solid obtained,
e) reslurrying at reflux the solid in a solvent selected from alcohols and polyols or mixtures thereof, in which the resulting mixture has an overall water content of between 2.5% and 0.01% by weight and
f) isolating the product.

In the method above described, the solvent in both the steps a) and e) is generally used in a ratio of between 50:1 and 2:1, preferably between 30:1 and 5:1, more preferably about 10:1, the ratio being expressed as ml of solvent per gram of moxifloxacin hydrochloride.

The mixture of moxifloxacin hydrochloride and solvent is kept under reflux for a variable period of time which will depend on various factors such as, for example, the type of solvent, the form of the starting product, the total quantity of water, etc., and is preferably at least 1 hour, more preferably about 4 hours.

The cooling of the mixture (step c) may be spontaneous or accelerated by appropriate means known to a person skilled in the art. The mixture may be cooled to room temperature or cooling may continue to lower temperatures; in general, it is preferred to allow the mixture to cool spontaneously until room temperature is reached.

In a particularly preferred method, cooling to room temperature takes place in about 2 hours and the mixture is allowed to rest at that temperature for a further 2 hours before the isolation (step d) is performed.

The product obtained is then filtered and charged again as wet in a reactor where a reslurry at reflux in one of the solvents as defined above is performed (step e), for a period of time ranging generally from 1 to 4 hours, preferably for about 2 hours. After cooling, the crystalline material is isolated (step f), generally by filtration, and dried under vacuum, preferably at 40°C for 12 hours, thus obtaining moxifloxacin hydrochloride form B.

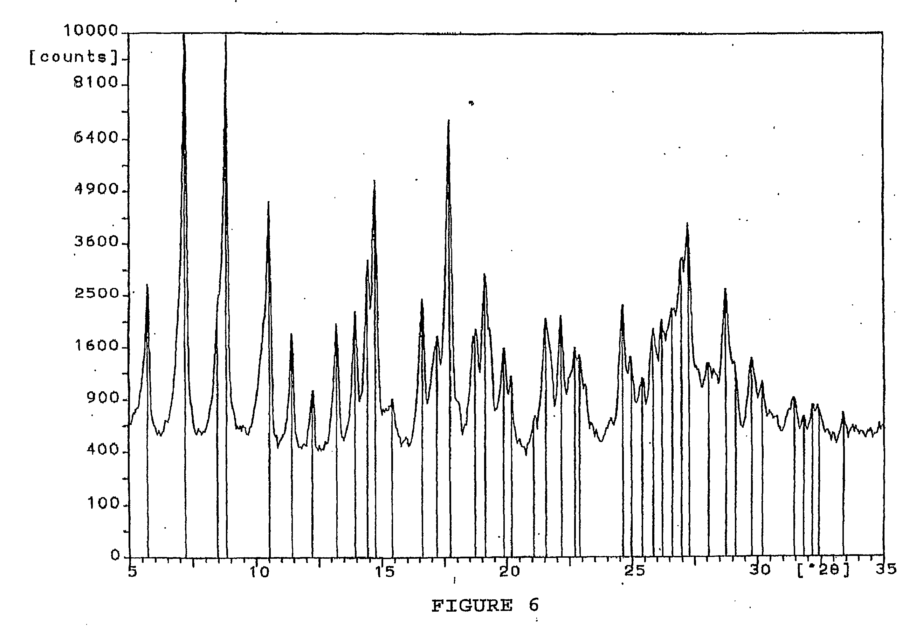

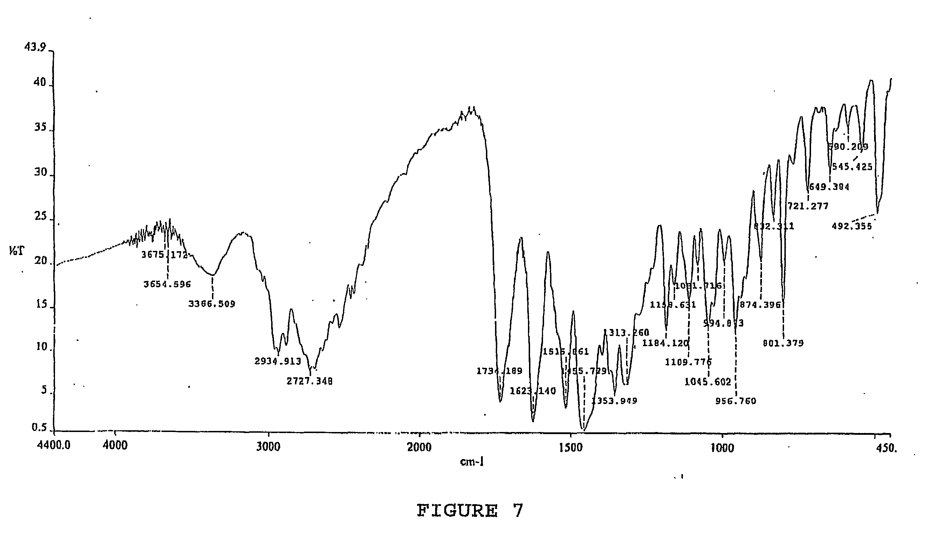

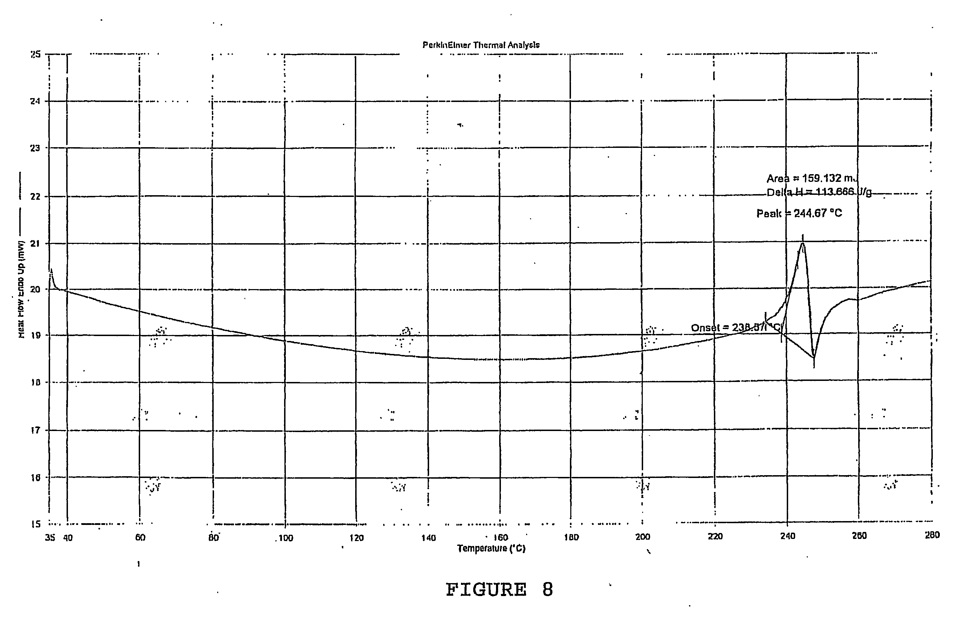

The novel crystalline form B of moxifloxacin hydrochloride is characterized by the X-ray diffraction spectrum (XRD) which is shown in Figure 6 and described in Table 3, by the IR spectrum which is given in Figure 7, and by the DSC trace which is shown in Figure 8. The differences in comparison with the known forms can clearly be distinguished by comparing this spectral data with that of the anhydrous and monohydrate forms described in US5849752. In particular, in the XRD spectrum of the novel form B, characteristic peaks situated at 7.2, 8.8, 10.5 and 14.7 are distinguished.

**Table 3**

| X-ray diffraction spectrum of moxifloxacin hydrochloride form B, registered on a Philips PW3710 spectrometer (X-ray Diffractometer). | | |
|---|---|---|
| (Cu Ka radiation, generator voltage 40 kV, slit divergence 1°, receiving slit 0.2 mm, scan mode step start angle 5.000, end angle 35.000, time per step 2.000s) | | |
| Angle (2Θ) | D (Å) | Rel. Intens. (I/I₀) |
| 5.700 | 15.4919 | 24.0 |
| 7.200 | 12.2675 | 100.0 |
| 8.470 | 10.4307 | 18.9 |
| 8.820 | 10.0176 | 91.6 |
| 10.505 | 8.4142 | 44.0 |
| 11.405 | 7.7522 | 14.6 |
| 12.220 | 7.2369 | 5.9 |
| 13.200 | 6.7018 | 16.2 |
| 13.925 | 6.3544 | 18.1 |
| 14.415 | 6.1395 | 26.6 |
| 14.740 | 6.0049 | 49.9 |
| 15.395 | 5.7508 | 4.9 |
| 16.600 | 5.3360 | 20.7 |
| 17.180 | 5.1571 | 13.7 |
| 17.705 | 5.0054 | 68.7 |
| 18.710 | 4.7387 | 13.7 |
| 19.105 | 4.6416 | 26.2 |
| 19.865 | 4.4657 | 11.8 |
| 20.155 | 4.4021 | 7.6 |
| 21.055 | 4.2159 | 2.4 |
| 21.545 | 4.1211 | 16.9 |
| 22.155 | 4.0090 | 17.3 |
| 22.690 | 3.9157 | 11.8 |
| 22.905 | 3.8794 | 10.5 |
| 24.610 | 3.6144 | 18.7 |
| 24.955 | 3.5652 | 10.0 |
| 25.385 | 3.5058 | 7.0 |
| 25.815 | 3.4483 | 14.5 |
| 26.195 | 3.3992 | 16.3 |
| 26.605 | 3.3477 | 18.4 |
| 26.960 | 3.3044 | 28.7 |
| 27.265 | 3.2681 | 37.0 |
| 28.045 | 3.1790 | 9.0 |
| 28.730 | 3.1047 | 22.2 |
| 29.110 | 3.0651 | 8.5 |
| 29.745 | 3.0011 | 9.6 |
| 30.170 | 2.9598 | 6.2 |
| 31.440 | 2.8430 | 4.1 |
| 31.795 | 2.8121 | 1.9 |
| 32.145 | 2.7823 | 3.1 |
| 32.410 | 2.7601 | 2.5 |
| 33.385 | 2.6817 | 1.8 |

The following preparation examples are now given to illustrate some of the methods that can be used to obtain the novel crystalline forms of moxifloxacin hydrochloride but they are not intended to limit the invention in any way.

### EXAMPLE 1

907.4 g of moxifloxacin hydrochloride monohydrate and 9070 ml of absolute ethanol (K.F. < 0.1%) were loaded into a 10 liter jacketed reactor and equipped with a mechanical stirrer, reflux condenser, and thermometer. The suspension was brought to reflux with stirring and was kept in those conditions for 4 hours. The temperature was then reduced to 20°C and the solid was filtered out and washed with 900 ml of absolute ethanol. The filtered solid was then discharged and dried under vacuum (30 mmHg) at 40°C for 18 hours to give moxifloxacin hydrochloride form A having a water content of about 1% (K.F.).

### EXAMPLE 2

10 g of anhydrous moxifloxacin hydrochloride and 200 ml of ethanol with 0.3% K.F. were loaded into a 250 ml flask. The mixture was brought to reflux and kept in those conditions for 4 hours, after which it was cooled to room temperature. The solid product was filtered out and washed with 30 ml of absolute ethanol. The solid was dried under vacuum (30 mmHg) at 40°C for 16 hours to give 8.5 g of moxifloxacin hydrochloride form A.

### EXAMPLE 3

20 g of anhydrous moxifloxacin hydrochloride and 250 ml of isopropanol with 0.2% K.F. were loaded into a 500 ml flask. The mixture was brought to reflux and was kept in those conditions for 4 hours, after which it was cooled to room temperature. The solid product was filtered out and washed with 50 ml of isopropanol. The solid was dried under vacuum (30 mmHg) at 40°C for 16 hours, to give 8.0 g of moxifloxacin hydrochloride form A.

### EXAMPLE 4

10 g of anhydrous moxifloxacin hydrochloride and 200 ml of isobutanol with 0.3% K.F. were loaded into a 250 ml flask. The mixture was brought to reflux and was kept in those conditions for 4 hours, after which it was cooled to room temperature. The solid product was filtered out and washed with 30 ml of absolute ethanol. The solid was dried under vacuum (30 mmHg) at 40°C for 16 hours to give 7.8 g of moxifloxacin hydrochloride form A.

### EXAMPLE 5

10 g of moxifloxacin hydrochloride monohydrate and 200 ml of 1,2-propandiol with 0.1% K.F. were loaded into a 250 ml flask. The mixture was brought to reflux and was kept in those conditions for 4 hours, after which it was cooled to room temperature. The solid product was filtered out and washed with 30 ml of absolute ethanol. The solid was dried under vacuum (30 mmHg) at 40°C for 16 hours to give 8.2 g of moxifloxacin hydrochloride form A.

### EXAMPLE 6

200 mg of moxifloxacin hydrochloride form A, 200 mg of Avicel Ph 112, and 5 mg of magnesium stearate were introduced into a mortar.

The powder was mixed and transferred into a Graseby Specac press and compressed by the application of a compression force of 5 tons. The procedure described was repeated a further 10 times, producing 10 identical tablets.

### EXAMPLE 7

10 g of anhydrous moxifloxacin hydrochloride and 200 m1 of ethanol with 0.3% K.F. were loaded into a 250 ml flask. The mixture was brought to reflux and kept in those conditions for 4 hours, after which it was cooled to room temperature. The solid product was filtered out and washed with 30 ml of absolute ethanol. The solid was recharged in a 250 ml flask, 200 ml of absolute ethanol with 0.1% K. F. were added and the mixture was brought to reflux and maintained in these conditions for 1 hour. Then the temperature was lowered to 25°C and the crystalline solid was filtered and washed with absolute ethanol. After drying under vacuum (30 mmHg) at 40°C for 16 hours 8.0 g of moxifloxacin hydrochloride form B were obtained.

### EXAMPLE 8

20 g of moxifloxacin hydrochloride monohydrate and 250 ml of isopropanol with 0.2% K.F. were loaded into a 500 ml flask. The mixture was brought to reflux and was kept in those conditions for 4 hours, after which it was cooled to room temperature. The solid product was filtered and washed with 50 ml of isopropanol. The solid was recharged in a 250 ml flask, 200 ml of isopropanol with 0.1% K. F. were added and the mixture was brought to reflux and maintained in these conditions for 1 hour. Then the temperature was lowered to 25°C and the crystalline solid was filtered and washed with isopropanol. After drying under vacuum (30 mmHg) at 40°C for 16 hours 17.2 g of moxifloxacin hydrochloride form B were obtained.

## Claims

1. Moxifloxacin hydrochloride form A, **characterized by** an X-ray diffraction spectrum having the following principal peaks:
| Angle (2Θ) | D (Å) | Rel. Intens. (I/I₀) |
|---|---|---|
| 5.815 | 15.1858 | 49.8 |
| 7.220 | 12.2335 | 100.0 |
| 8.575 | 10.3032 | 86.1 |
| 10.335 | 8.5522 | 87.2 |
| 12.310 | 7.1842 | 19.4 |
| 13.200 | 6.7018 | 17.0 |
| 14.085 | 6.2826 | 16.3 |
| 14.535 | 6.0891 | 11.1 |
| 14.870 | 5.9527 | 20.6 |
| 15.185 | 5.8299 | 17.6 |
| 15.675 | 5.6487 | 1.9 |
| 16.620 | 5.3296 | 18.3 |
| 17.335 | 5.1114 | 60.1 |
| 17.850 | 4.9650 | 80.9 |
| 19.315 | 4.5916 | 53.7 |
| 19.760 | 4.4892 | 19.1 |
| 20.375 | 4.3551 | 2.5 |
| 21.640 | 4.1033 | 47.6 |
| 22.295 | 3.9842 | 12.7 |
| 23.160 | 3.8373 | 4.2 |
| 23.625 | 3.7628 | 1.9 |
| 24.705 | 3.6007 | 26.9 |
| 25.115 | 3.5428 | 17.6 |
| 25.815 | 3.4483 | 15.6 |
| 26.440 | 3.3682 | 39.4 |
| 27.365 | 3.2564 | 36.3 |
| 27.970 | 3.1874 | 17.8 |
| 28.360 | 3.1444 | 14.5 |
| 29.015 | 3.0749 | 28.2 |
| 29.965 | 2.9795 | 13.9 |
| 30.545 | 2.9243 | 4.8 |
| 31.575 | 2.8312 | 5.9 |
| 32.270 | 2.7718 | 12.2 |
| 33.900 | 2.6421 | 6.4 |

2. Moxifloxacin hydrochloride form A, **characterized by** an X-ray diffraction spectrum as shown in Figure 1.

3. Moxifloxacin hydrochloride form A, **characterized by** a solid-state ¹³C-NMR spectrum as shown in Figure 2.

4. Moxifloxacin hydrochloride form A, **characterized by** an IR spectrum as shown in Figure 3.

5. Moxifloxacin hydrochloride form A, **characterized by** a DSC graph as shown in Figure 4.

6. A method for the preparation of moxifloxacin hydrochloride form, A which comprises the steps of:
a) suspending moxifloxacin hydrochloride in a solvent selected from alcohols and polyols or mixtures thereof, in which the resulting mixture has an overall water content of between 2.5% and 0.01% by weight,
b) heating the mixture under reflux,
c) cooling, and
d) isolating the product.

7. A method according to Claim 6 in which the moxifloxacin hydrochloride in step a) is in anhydrous or monohydrate crystalline form.

8. A method according to Claim 7 in which the moxifloxacin hydrochloride is in an anhydrous form having a water content of less than 0.3%.

9. A method according to Claim 6 in which the solvent is a C₁-C₆ alcohol or polyol, preferably ethanol or isopropanol.

10. A method according to Claim 6 in which the solvent has a water content of between 1% and 0.01%, preferably between 0.3% and 0.01%, more preferably between 0.1% and 0.01%.

11. A method according to Claim 6 in which the mixture is cooled to room temperature.

12. A method according to Claim 6 in which the solvent is used in a ratio of between 50:1 and 2:1, preferably between 30:1 and 5:1, more preferably about 10:1, the ratio being expressed as ml of solvent per gram of moxifloxacin hydrochloride.

13. A method according to Claim 6 in which the mixture is heated under reflux for at least 1 hour, preferably for about 4 hours.

14. Moxifloxacin hydrochloride form A according to claims 1 to 5 for use as a medicament.

15. Use of moxifloxacin hydrochloride form A, according to claims 1 to 5, for manufacturing a medicament for the treatment of bacterial infections.

16. Pharmaceutical compositions comprising moxifloxacin hydrochloride form A according to Claims 1 to 5 and at least one pharmaceutically acceptable excipient.

17. Moxifloxacin hydrochloride form B, **characterized by** an X-ray diffraction spectrum having the following principal peaks:
| Angle (2Θ) | D (Å) | Rel. Intens. (I/I₀) |
|---|---|---|
| 5.700 | 15.4919 | 24.0 |
| 7.200 | 12.2675 | 100.0 |
| 8.470 | 10.4307 | 18.9 |
| 8.820 | 10.0176 | 91.6 |
| 10.505 | 8.4142 | 44.0 |
| 11.405 | 7.7522 | 14.6 |
| 12.220 | 7.2369 | 5.9 |
| 13.200 | 6.7018 | 16.2 |
| 13.925 | 6.3544 | 18.1 |
| 14.415 | 6.1395 | 26.6 |
| 14.740 | 6.0049 | 49.9 |
| 15.395 | 5.7508 | 4.9 |
| 16.600 | 5.3360 | 20.7 |
| 17.180 | 5.1571 | 13.7 |
| 17.705 | 5.0054 | 68.7 |
| 18.710 | 4.7387 | 13.7 |
| 19.105 | 4.6416 | 26.2 |
| 19.865 | 4.4657 | 11.8 |
| 20.155 | 4.4021 | 7.6 |
| 21.055 | 4.2159 | 2.4 |
| 21.545 | 4.1211 | 16.9 |
| 22.155 | 4.0090 | 17.3 |
| 22.690 | 3.9157 | 11.8 |
| 22.905 | 3.8794 | 10.5 |
| 24.610 | 3.6144 | 18.7 |
| 24.955 | 3.5652 | 10.0 |
| 25.385 | 3.5058 | 7.0 |
| 25.815 | 3.4483 | 14.5 |
| 26.195 | 3.3992 | 16.3 |
| 26.605 | 3.3477 | 18.4 |
| 26.960 | 3.3044 | 28.7 |
| 27.265 | 3.2681 | 37.0 |
| 28.045 | 3.1790 | 9.0 |
| 28.730 | 3.1047 | 22.2 |
| 29.110 | 3.0651 | 8.5 |
| 29.745 | 3.0011 | 9.6 |
| 30.170 | 2.9598 | 6.2 |
| 31.440 | 2.8430 | 4.1 |
| 31.795 | 2.8121 | 1.9 |
| 32.145 | 2.7823 | 3.1 |
| 32.410 | 2.7601 | 2.5 |
| 33.385 | 2.6817 | 1.8 |

18. Moxifloxacin hydrochloride form B, **characterized by** an X-ray diffraction spectrum as shown in Figure 6.

19. Moxifloxacin hydrochloride form B, **characterized by** an IR spectrum as shown in Figure 7.

20. Moxifloxacin hydrochloride form B, **characterized by** a DSC graph as shown in Figure 8.

21. A method for the preparation of moxifloxacin hydrochloride form B, which comprises the steps the steps of:
a) suspending moxifloxacin hydrochloride in a solvent selected from alcohols and polyols or mixtures thereof, in which the resulting mixture has an overall water content of between 2.5% and 0.01% by weight,
b) heating the mixture under reflux,
c) cooling,
d) isolating the product,
e) reslurrying at reflux the solid in a solvent selected from alcohols and polyols or mixtures thereof, in which the resulting mixture has an overall water content of between 2.5% and 0.01% by weight and
f) isolating the product.

22. A method according to Claim 21 in which the moxifloxacin hydrochloride in step a) is in anhydrous or monohydrate crystalline form.

23. A method according to Claim 22 in which the moxifloxacin hydrochloride is in an anhydrous form having a water content of less than 0.3%.

24. A method according to Claim 21 in which the solvent of steps a) and e) is a C₁-C₆ alcohol or polyol, preferably ethanol or isopropanol.

25. A method according to Claim 21 in which the solvent of steps a) and e) has a water content of between 1% and 0.01%, preferably between 0.3% and 0.01%, more preferably between 0.1% and 0.01%.

26. A method according to Claim 21 in which the mixture is cooled to room temperature.

27. A method according to Claim 21 in which the solvent is used in a ratio of between 50:1 and 2:1, preferably between 30:1 and 5:1, more preferably about 10:1, the ratio being expressed as ml of solvent per gram of moxifloxacin hydrochloride.

28. A method according to Claim 21 in which step e) is performed by heating the mixture under reflux for 1 to 4 hours, preferably for about 2 hours.

29. Moxifloxacin hydrochloride form B according to claims 18 to 21, for use as a medicament.

30. Use of moxifloxacin hydrochloride form B according to claims 18 to 21 for manufacturing a medicament for the treatment of bacterial infections.

31. Pharmaceutical compositions comprising moxifloxacin hydrochloride form B according to Claims 16 to 19 and at least one pharmaceutically acceptable excipient.

## Patentansprüche

1. Moxifloxacin-Hydrochlorid Form A, **gekennzeichnet durch** ein Röntgenbeugungsspektrum, das die folgenden Hauptpeaks aufweist:
| Winkel (2Θ) | D (Å) | Rel. Intens. (I/I₀) |
|---|---|---|
| 5.815 | 15.1858 | 49.8 |
| 7.220 | 12.2335 | 100.0 |
| 8.575 | 10.3032 | 86.1 |
| 10.335 | 8.5522 | 87.2 |
| 12.310 | 7.1842 | 19.4 |
| 13.200 | 6.7018 | 17.0 |
| 14.085 | 6.2826 | 16.3 |
| 14.535 | 6.0891 | 11.1 |
| 14.870 | 5.9527 | 20.6 |
| 15.185 | 5.8299 | 17.6 |
| 15.675 | 5.6487 | 1.9 |
| 16.620 | 5.3296 | 18.3 |
| 17.335 | 5.1114 | 60.1 |
| 17.850 | 4.9650 | 80.9 |
| 19.315 | 4.5916 | 53.7 |
| 19.760 | 4.4892 | 19.1 |
| 20.375 | 4.3551 | 2.5 |
| 21.640 | 4.1033 | 47.6 |
| 22.295 | 3.9542 | 12.7 |
| 23.160 | 3.8373 | 4.2 |
| 23.625 | 3.7628 | 1.9 |
| 24.705 | 3.6007 | 26.9 |
| 25.115 | 3.5428 | 17.6 |
| 25.815 | 3.4483 | 15.6 |
| 26.440 | 3.3682 | 39.4 |
| 27.365 | 3.2564 | 36.3 |
| 27.970 | 3.1874 | 17.8 |
| 23.360 | 3.1444 | 14.5 |
| 29.015 | 3.0749 | 28.2 |
| 29.965 | 2.9795 | 13.9 |
| 30.545 | 2.9243 | 4.8 |
| 31.575 | 2.8312 | 5.9 |
| 32.270 | 2.7718 | 12.2 |
| 33.300 | 2.6421 | 6.4 |

2. Moxifloxacin-Hydrochlorid Form A, **gekennzeichnet durch** ein wie in Figur 1 gezeigtes Röntgenbeugungsspektrum.

3. Moxifloxacin-Hydrochlorid Form A, **gekennzeichnet durch** ein wie in Figur 2 gezeigtes ¹³C-Festkörper-NMR-Spektrum.

4. Moxifloxacin-Hydrochlorid Form A, **gekennzeichnet durch** ein wie in Figur 3 gezeigtes IR-Spektrum.

5. Moxifloxacin-Hydrochlorid Form A, **gekennzeichnet durch** einen wie in Figur 4 gezeigten DSC-Graphen.

6. Verfahren zur Herstellung von Moxifloxacin-Hydrochlorid Form A, umfassend die Schritte:
a) Suspendieren von Moxifloxacin-Hydrochlorid in einem aus Alkoholen und Polyolen oder Mischungen davon ausgewählten Lösungsmittel, wobei die resultierende Mischung einen Gesamtwassergehalt von zwischen 2,5 Gew.-% und 0,01 Gew.-% aufweist,
b) Erwärmten der Mischung unter Rückfluss,
c) Kühlen, und
d) Isolieren des Produkts.

7. Verfahren gemäß Anspruch 6, wobei das Moxifloxacin-Hydrochlorid in Schritt a) in wasserfreier oder monohydratischer, kristalliner Form vorliegt.

8. Verfahren gemäß Anspruch 7, wobei das Moxifloxacin-Hydrochlorid in einer wasserfreien Form vorliegt, die einen Wassergehalt von weniger als 0,3% aufweist.

9. Verfahren gemäß Anspruch 6, wobei das Lösungsmittel ein C₁-C₆-Alkohol oder Polyol, bevorzugt Ethanol oder Isopropanol ist.

10. Verfahren gemäß Anspruch 6, wobei das Lösungsmittel einen Wassergehalt von zwischen 1% und 0,01%, bevorzugt zwischen 0,3% und 0,01%, weiter bevorzugt zwischen 0,1% und 0,01% aufweist.

11. Verfahren gemäß Anspruch 6, wobei die Mischung auf Raumtemperatur gekühlt wird.

12. Verfahren gemäß Anspruch 6, wobei das Lösungsmittel in einem Verhältnis von zwischen 50:1 und 2:1, bevorzugt zwischen 30:1 und 5:1, weiter bevorzugt etwa 10:1 verwendet wird, wobei das Verhältnis als ml an Lösungsmittel pro Gramm Moxifloxacin-Hydrochlorid ausgedrückt wird.

13. Verfahren gemäß Anspruch 6, wobei die Mischung mindestens 1 Stunde, bevorzugt etwa 4 Stunden unter Rückfluss erwärmt wird.

14. Moxifloxacin-Hydrochlorid Form A gemäß den Ansprüchen 1 bis 5 zur Verwendung als Arzneimittel.

15. Verwendung von Moxifloxacin-Hydrochlorid Form A gemäß den Ansprüchen 1 bis 5 zur Herstellung eines Arzneimittels zur Behandlung bakterieller Infektionen.

16. Pharmazeutische Zusammensetzungen, die Moxifloxacin-Hydrochlorid Form A gemäß den Ansprüchen 1 bis 5 und mindestens einen pharmazeutisch verträglichen Hilfsstoff umfassen.

17. Moxifloxacin-Hydrochlorid Form B **gekennzeichnet durch** ein Röntgenbeugungsspektrum, das die folgenden Hauptpeaks aufweist:
| Winkel (2Θ) | D (Å) | Rel. Intens. (I/I₀) |
|---|---|---|
| 5.700 | 15.4319 | 24.0 |
| 7.200 | 12.2675 | 100.0 |
| 8.470 | 10.4307 | 18.9 |
| 8.820 | 10.0176 | 91.6 |
| 10.505 | 8.4142 | 44.0 |
| 11.405 | 7.7522 | 14.6 |
| 12.220 | 7.2369 | 5.9 |
| 13.200 | 6.7018 | 16.2 |
| 13.925 | 6.3544 | 18.1 |
| 14.415 | 6.1395 | 26.6 |
| 14.740 | 6.0049 | 49.9 |
| 15.395 | 5.7508 | 4.9 |
| 16.600 | 5.3360 | 20.7 |
| 17.180 | 5.1571 | 13.7 |
| 17.705 | 5.0054 | 68.7 |
| 18.710 | 4.7387 | 13.7 |
| 19.105 | 4.6416 | 26.2 |
| 19.865 | 4.4657 | 11.8 |
| 20.155 | 4.4021 | 7.6 |
| 21.055 | 4.2159 | 2.4 |
| 21.545 | 4.1211 | 16.9 |
| 22.155 | 4.0090 | 17.3 |
| 22.690 | 3.9157 | 11.8 |
| 22.905 | 3,8754 | 10.5 |
| 24.610 | 3.6144 | 18.7 |
| 24.955 | 3.5652 | 10.0 |
| 25.385 | 3.5058 | 7.0 |
| 25.815 | 3.4483 | 14.5 |
| 26.195 | 3.3992 | 16.3 |
| 26.605 | 3.3477 | 18.4 |
| 26.960 | 3.3044 | 20.7 |
| 27.265 | 3.2681 | 37.0 |
| 28.045 | 3.1790 | 9.0 |
| 28.730 | 3.1047 | 22.2 |
| 29.110 | 3.0651 | 8.5 |
| 29.745 | 3.0011 | 9.6 |
| 30.170 | 2.9593 | 6.2 |
| 31.440 | 2.8430 | 4.1 |
| 31.795 | 2.8121 | 1.9 |
| 32.145 | 2.7823 | 3.1 |
| 32.410 | 2.7601 | 2.5 |
| 33.385 | 2.6817 | 1.8 |

18. Moxifloxacin-Hydrochlorid Form B, **gekennzeichnet durch** ein wie in Figur 6 gezeigtes Röntgenbeugungsspektrum.

19. Moxifloxacin-Hydrochlorid Form B, **gekennzeichnet durch** ein wie in Figur 7 gezeigtes IR-Spektrum.

20. Moxifloxacin-Hydrochlorid Form B, **gekennzeichnet durch** einen wie in Figur 8 gezeigten DSC-Graphen.

21. Verfahren zur Herstellung von Moxifloxacin-Hydrochlorid Form B, umfassend die Schritte:
a) Suspendieren von Moxifloxacin-Hydrochlorid in einem aus Alkoholen und Polyolen oder Mischungen davon ausgewählten Lösungsmittel, wobei die resultierende Mischung einen Gesamtwassergehalt von zwischen 2,5 Gew.-% und 0,01 Gew.-% aufweist,
b) Erwärmen der Mischung unter Rückfluss,
c) Kühlen,
d) Isolieren des Produkts,
e) Wiederaufschlämmen des Feststoffs bei Rückfluss in einem aus Alkoholen und Polyolen oder Mischungen davon ausgewählten Lösungsmittel, wobei die resultierende Mischung einen Gesamtwassergehalt von zwischen 2,5 Gew.-% und 0,01 Gew.-% aufweist, und
(f) Isolieren des Produkts.

22. Verfahren gemäß Anspruch 21, wobei das Moxifloxacin-Hydrochlorid in Schritt a) in wasserfreier oder monohydratischer, kristalliner Form vorliegt.

23. Verfahren gemäß Anspruch 22, wobei das Moxifloxacin-Hydrochlorid in einer wasserfreien Form vorliegt, die einen Wassergehalt von weniger als 0,3% aufweist.

24. Verfahren gemäß Anspruch 21, wobei das Lösungsmittel von den Schritten a) und e) ein C₁-C₆-Alkohol oder Polyol, bevorzugt Ethanol oder Isopropanol ist.

25. Verfahren gemäß Anspruch 21, wobei das Lösungsmittel von den Schritten a) und e) einen Wassergehalt von zwischen 1% und 0,01%, bevorzugt zwischen 0,3% und 0,01%, weiter bevorzugt zwischen 0,1% und 0,01% aufweist.

26. Verfahren gemäß Anspruch 21, wobei die Mischung auf Raumtemperature gekühlt wird.

27. Verfahren gemäß Anspruch 21, wobei das Lösungsmittel in einem Verhältnis von zwischen 50:1 und 2:1, bevorzugt zwischen 30:1 und 5:1, weiter bevorzugt etwa 10:1 verwendet wird, wobei das Verhältnis als ml an Lösungsmittel pro Gramm Moxifloxacin-Hydrochlorid ausgedrückt wird.

28. Verfahren gemäß Anspruch 21, wobei Schritt e) durch Erwärmen der Mischung unter Rückfluss für 1 bis 4 Stunden, bevorzugt für etwa 2 Stunden durchgeführt wird.

29. Moxifloxacin-Hydrochlorid Form B gemäß den Ansprüchen 18 bis 21 zur Verwendung als Arzneimittel.

30. Verwendung von Moxifloxacin-Hydrochlorid Form B gemäß den Ansprüchen 18 bis 21 zur Herstellung eins Arzneimittels zur Behandlung bakterieller Infektionen.

31. Pharmazeutische Zusammensetzungen, die Moxifloxacin-Hydrochlorid Form 8 gemäß den Ansprüchen 16 bis 19 und mindestens einen pharmazeutisch verträglichen Hilfsstoff umfassen.

## Revendications

1. Chlorhydrate de moxifloxacine de forme A, **caractérisé par** un spectre de diffraction de rayons X ayant les pics principaux suivants :
| Angle (2Θ) | D (Å) | Intensité relative (I/I₀) |
|---|---|---|
| 5,815 | 15,1858 | 49,8 |
| 7,220 | 12,2335 | 100,0 |
| 8,575 | 10,3032 | 86,1 |
| 10.335 | 8,5522 | 87,2 |
| 12,310 | 7,1842 | 19,4 |
| 13,200 | 6,7018 | 17,0 |
| 14,085 | 6,2826 | 16,3 |
| 14,535 | 6,0891 | 11,1 |
| 14,870 | 5,9527 | 20,6 |
| 15,185 | 5,8299 | 17,6 |
| 15,675 | 5,6487 | 1,9 |
| 16,620 | 5,3296 | 18.3 |
| 17,335 | 5,1114 | 60,1 |
| 17,850 | 4,9650 | 80,9 |
| 19,315 | 4,5916 | 53,7 |
| 19,760 | 4,4892 | 19,1 |
| 20,375 | 4,3551 | 2,5 |
| 21,640 | 4,1033 | 47,6 |
| 22,295 | 3,9842 | 12,7 |
| 23,160 | 3,8373 | 4,2 |
| 23,625 | 3,7628 | 1,9 |
| 24,705 | 3,6007 | 26,9 |
| 25,115 | 3,5428 | 17,6 |
| 25,815 | 3,4483 | 15,6 |
| 26,440 | 3,3682 | 39,4 |
| 27,365 | 3,2564 | 36,3 |
| 27,970 | 3,1874 | 17,8 |
| 28,360 | 3,1444 | 14,5 |
| 29,015 | 3,0749 | 28,2 |
| 29,965 | 2,9795 | 13,9 |
| 30,545 | 2,9243 | 4,8 |
| 31,575 | 2,8312 | 5,9 |
| 32,270 | 2,7718 | 12,2 |
| 33,900 | 2,6421 | 6,4 |

2. Chlorhydrate de moxifloxacine de forme A, **caractérisé par** le spectre de diffraction de rayons X montré sur la figure 1.

3. Chlorhydrate de moxifloxacine de forme A, **caractérisé par** le spectre RMN de C¹³ à l'état solide montré sur la figure 2.

4. Chlorhydrate de moxifloxacine de forme A, **caractérisé par** le spectre IR montré sur la figure 3.

5. Chlorhydrate de moxifloxacine de forme A, **caractérisé par** le graphique de calorimétrie à balayage différentiel (DSC de l'anglais Differential Scanning Calorimetry) montré sur la figure 4.

6. Procédé de préparation de chlorhydrate de moxifloxacine de forme A qui comprend les étapes consistant à :
a) mettre en suspension le chlorhydrate de moxifloxacine dans un solvant choisi parmi des alcools et des polyols ou des mélanges de ceux-ci, le mélange obtenu ayant une teneur en eau globale comprise entre 2,5 % et 0,01 % en poids,
b) chauffer le mélange à reflux,
c) refroidir, et
d) isoler le produit.

7. Procédé selon la revendication 6, dans lequel le chlorhydrate de moxifloxacine dans l'étape a) se présente sous forme cristalline anhydre ou monohydratée.

8. Procédé selon la revendication 7, dans lequel le chlorhydrate de moxifloxacine se présente sous une forme anhydre ayant une teneur en eau inférieure à 0,3%.

9. Procédé selon la revendication 6, dans lequel le solvant est un alcool ou un polyol en C₁-C₆, de préférence l'éthanol ou l'alcool isopropylique.

10. Procédé selon la revendication 6, dans lequel le solvant a une teneur en eau comprise entre 1 % et 0,01 %, de préférence entre 0,3 % et 0.01 %, de manière davantage préférée entre 0,1 % et 0,01 %.

11. Procédé selon la revendication 6, dans lequel le mélange est refroidi à la température ambiante.

12. Procédé selon la revendication 6, dans lequel le solvant est utilisé selon un rapport compris entre 50/1 et 2/1, de préférence entre 30/1 et 5/1, de manière davantage préférée d'environ 10/1, le rapport étant exprimé en ml de solvant par gramme de chlorhydrate de moxifloxacine.

13. Procédé selon la revendication 6, dans lequel le mélange est chauffé à reflux pendant au moins 1 heure, de préférence pendant environ 4 heures.

14. Chlorhydrate de moxifloxacine de forme A selon les revendications 1 à 5 destiné à être utilisé comme médicament.

15. Utilisation de chlorhydrate de moxifloxacine de forme A selon les revendications 1 à 5 pour la fabrication d'un médicament en vue du traitement d'infections bactériennes.

16. Compositions pharmaceutiques comprenant du chlorhydrate de moxifloxacin de forme A selon les revendications 1 à 5 et au moins un excipient acceptable sur le plan pharmaceutique.

17. Chlorhydrate de moxifloxacine de forme B, **caractérisé par** un spectre de diffraction de rayons X ayant les pics principaux suivants :
| Angle (2Θ) | D (Å) | Intensité relative (I/I₀) |
|---|---|---|
| 5,700 | 15,4919 | 24,0 |
| 7,200 | 12,2675 | 100,0 |
| 8,470 | 10,4307 | 18,9 |
| 8,820 | 10,0176 | 91,6 |
| 10,505 | 8,4142 | 44,0 |
| 11,405 | 7,7522 | 14,6 |
| 12,220 | 7,2369 | 5,9 |
| 13,200 | 6,7018 | 16,2 |
| 13,925 | 6,3544 | 18,1 |
| 14,415 | 6,1395 | 26,6 |
| 14,740 | 6,0049 | 49,9 |
| 15,395 | 5,7508 | 4,9 |
| 16,600 | 5,3360 | 20,7 |
| 17,180 | 5,1571 | 13,7 |
| 17,705 | 5,0054 | 68,7 |
| 18,710 | 4,7387 | 13,7 |
| 19,105 | 4,6416 | 26,2 |
| 19,865 | 4,4657 | 11,8 |
| 20,155 | 4,4021 | 7.6 |
| 21,055 | 4,2159 | 2,4 |
| 21,545 | 4,1211 | 16,9 |
| 22,155 | 4,0090 | 17,3 |
| 22,690 | 3,9157 | 11,8 |
| 22,905 | 3,8794 | 10,5 |
| 24,610 | 3,6144 | 18,7 |
| 24,955 | 3,5652 | 10,0 |
| 25,385 | 3,5058 | 7,0 |
| 25,815 | 3,4483 | 14,5 |
| 26,195 | 3,3992 | 16,3 |
| 26,605 | 3,3477 | 18,4 |
| 26,960 | 3,3044 | 28,7 |
| 27,265 | 3,2681 | 37,0 |
| 28,045 | 3,1790 | 9,0 |
| 28,730 | 3,1047 | 22,2 |
| 29,110 | 3,0651 | 8,5 |
| 29,745 | 3,0011 | 9,6 |
| 30,170 | 2,9598 | 6,2 |
| 31,440 | 2,8430 | 4,1 |
| 31,795 | 2,8121 | 1,9 |
| 32,145 | 2,7823 | 3,1 |
| 32,410 | 2,7601 | 2,5 |
| 33,385 | 2,6817 | 1,8 |

18. Chlorhydrate de moxifloxacine de forme B, **caractérisé par** le spectre de diffraction de rayons X montré sur la figure 6.

19. Chlorhydrate de moxifloxacine de forme B, **caractérisé par** le spectre IR montré sur la figure 7.

20. Chlorhydrate de moxifloxacine de forme B, **caractérisé par** le graphique de calorimétrie à balayage différentiel (DSC de l'anglais Differential Scanning Calorimetry) montré sur la figure 8.

21. Procédé de préparation de chlorhydrate de moxifloxacine de forme B, qui comprend les étapes consistant à :
a) mettre en suspension le chlorhydrate de moxifloxacine dans un solvant choisi parmi des alcools et des polyols ou des mélanges de ceux-ci, le mélange obtenu ayant une teneur en eau globale comprise entre 2,5 % et 0,01 % en poids,
b) chauffer le mélange à reflux,
c) refroidir,
d) isoler le produit,
e) remettre en suspension à reflux le solide dans un solvant choisi parmi des alcools et des polyols ou des mélanges de ceux-ci, le mélange obtenu ayant une teneur en eau globale comprise entre 2,5 % et 0,01 % en poids, et
f) à isoler le produit.

22. Procédé selon la revendication 21, dans lequel le chlorhydrate de moxifloxacine dans l'étape a) se présente sous forme cristalline anhydre ou monohydratée.

23. Procédé selon la revendication 22, dans lequel le chlorhydrate de moxifloxacine se présente sous une forme anhydre ayant une teneur en eau inférieure à 0,3%.

24. Procédé selon la revendication 21, dans lequel le solvant des étapes a) et e) est un alcool ou un polyol en C₁-C₆, de préférence l'éthanol ou l'alcool isopropylique.

25. Procédé selon la revendication 21, dans lequel le solvant des étapes a) et e) a une teneur en eau comprise entre 1 % et 0,01 %, de préférence entre 0,3 % et 0,01 %, de manière davantage préférée entre 0,1 % et 0,01 %.

26. Procédé selon la revendication 21, dans lequel le mélange est refroidi à la température ambiante.

27. Procédé selon la revendication 21, dans lequel le solvant est utilisé selon un rapport compris entre 50/1 et 2/1, de préférence entre 30/1 et 5/1, de manière davantage préférée d'environ 10/1, le rapport étant exprimé en ml de solvant par gramme de chlorhydrate de moxifloxacine.

28. Procédé selon la revendication 21, dans lequel l'étape e) est effectuée en chauffant le mélange à reflux pendant 1 à 4 heures, de préférence pendant environ 2 heures.

29. Chlorhydrate de moxifloxacine de forme B selon les revendications 18 à 21, destiné à être utilisé comme médicament.

30. Utilisation de chlorhydrate de moxifloxacine de forme B selon les revendications 18 à 21 pour la fabrication d'un médicament en vue du traitement d'infections bactériennes.

31. Compositions pharmaceutiques comprenant du chlorhydrate de moxifloxacine de forme B selon les revendications 16 à 19 et au moins un excipient pharmaceutiquement acceptable.
